(19) Europäisches Patentamt
European Patent Office
Office européen des brevets

(11) Veröffentlichungsnummer: **0 054 509**
**B1**

(12) # EUROPÄISCHE PATENTSCHRIFT

(45) Veröffentlichungstag der Patentschrift: **09.04.86**

(21) Anmeldenummer: **81810431.7**

(22) Anmeldetag: **29.10.81**

(51) Int. Cl.[4]: **C 08 K 5/42**, C 08 G 59/68, C 08 G 65/10, C 08 L 61/00, C 08 F 2/50, C 07 C 147/14, C 07 C 147/00

(54) **Sulfoxoniumsalzpolymerisationskatalysatoren.**

(30) Priorität: **04.11.80 GB 8035382**

(43) Veröffentlichungstag der Anmeldung: **23.06.82 Patentblatt 82/25**

(45) Bekanntmachung des Hinweises auf die Patenterteilung: **09.04.86 Patentblatt 86/15**

(84) Benannte Vertragsstaaten: **BE CH DE FR GB IT LI NL SE**

(56) Entgegenhaltungen:
**TETRAHEDRON LETTERS, Nr. 31, August 1966, Seiten 3681-3687, Oxford, G.B., W.E. TRUCE et al.: "Sulfone-stabilized oxosulfonium ylides"**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Green, George Edward, Dr., 18 Church Street, Stapleford Cambridge CB2 5DS (GB)**
Erfinder: **Irving, Edward, Dr., 41, Swaffham Road, Burwell Cambridge CB5 0AN (GB)**
Erfinder: **Stark, Bernard Peter, Dr., 41 High Street, Great Shelford Cambridge CB2 5EH (GB)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

EP 0 054 509 B1

**Beschreibung**

Vorliegende Erfindung betrifft Sulfoxoniumsalze und einen kationisch polymerisierbaren Stoff sowie ein Sulfoxoniumsalz enthaltende Zusammensetzungen. Ferner bezieht sie sich auf die Polymerisation solcher Zusammensetzungen mittels aktinischer Strahlung, die wahlweise weitere Vernetzung so erhaltener photopolymerisierter Produkte mittels Hitze in Gegenwart von Heisshärtern, sowie die Polymerisation solcher Zusammensetzungen unter der Einwirkung, von Hitze allein.

Aus verschiedenen Gründen hat es sich als wünschenswert erwiesen, die Polymerisation organischer Materialien mittels aktinischer Strahlung zu induzieren. Bei Anwendung von Photopolymerisationsmethoden kann man beispielsweise die Verwendung organischer Lösungsmittel mit den damit verbundenen Risiken der Giftigkeit, Entflammbarkeit und Umweltverschmutzung und den Kosten der Lösungsmittelrückgewinnung vermeiden. Die Photopolymerisation ermöglicht es, dass nur definierte Stellen der Harzzusammensetzung, d.h. solche, die bestrahlt wurden, unlöslich gemacht werden und gestattet somit die Herstellung von gedruckten Schaltungen und Druckplatten oder erlaubt es, die Verklebung von Substraten auf die erforderlichen Zonen zu beschränken. Bei Produktionsverfahren sind ferner Bestrahlungsmethoden häufig schneller als solche, die eine Erhitzung und nachfolgende Kühlstufe einschliessen.

Es ist seit Jahren bekannt, dass gewisse aromatische Diazoniumsalze bei der Belichtung mit aktinischer Strahlung einer Zersetzung unterliegen und dass, wenn das Salz mit einer kationisch polymerisierbaren Substanz gemischt ist, die bei der Bestrahlung in situ erzeugte Lewis-Säure dann eine Polymerisation induziert (siehe z. B. britische Patentschrift Nr. 1 321 263). Die Diazoniumsalze sind jedoch nicht völlig befriedigend: die Topfzeit des Gemischs aus Diazoniumsalz und kationisch polymerisierbarer Substanz ist häufig zu kurz, insbesondere im Tageslicht, und zweitens entsteht Stickstoff bei der Freisetzung des Lewis-Säurekatalysators, wobei die Gasentwicklung den Verfahrensbereich, in dem die Katalysatoren erfolgreich eingesetzt werden können, beschränkt.

Es wurden deshalb zahlreiche Vorschläge für den Ersatz dieser Diazoniumsalze durch andere Salze gemacht, die bei der Freisetzung eines Säurekatalysators unter Bestrahlung nicht auch Stickstoff entwickeln: die Oniumsalze des Schwefels und Jodoniumsalze wurden besonders intensiv studiert.

So ist es kürzlich aus der britischen Patentschrift Nr. 1 516 511 und der parallelen U.S. Patentschrift Nr. 4 058 401 bekannt geworden, dass ein Mono-1,2-epoxid, ein Epoxidharz (d.h. eine Substanz, die im Durchschnitt mehr als eine 1,2-Epoxidgruppe enthält), oder deren Gemisch mittels eines strahlungsempfindlichen aromatischen Oniumsalzes des Sauerstoffs, Schwefels, Selens oder Tellurs, das in einer Menge vorliegt, die fähig ist, die Polymerisation oder Härtung des Epoxids (bzw. Polyepoxids) durch Freisetzung eines Brønsted-Säurekatalysators bei der Belichtung mit Strahlungsenergie zu bewirken, polymerisiert oder gehärtet werden kann.

In der britischen Patentschrift Nr. 1 518 141 wird beschrieben, dass monomere und präpolymere, kationisch polymerisierbare organische Materialien, die frei von 1,2-Epoxidgruppen und unter Vinylmonomeren, Vinylpräpolymeren, cyclischen Aethern, cyclischen Estern, cyclischen Sulfiden, cyclischen Aminen und cyclischen Organosiliciumverbindungen ausgewählt sind, ebenfalls durch Belichtung mit Strahlungsenergie in Gegenwart dieser Oniumsalze polymerisiert werden können.

Aus der US. Patentschrift Nr. 4 102 687 ist bekannt geworden, dass man die Härtung von Harnstoff/Formaldehydharzen, Melamin/Formaldehydharzen und Phenol/Formaldehydharzen durch Belichtung mit ultravioletter 8trahlung in Gegenwart eines Oniumsalzes auslösen kann, wobei die Härtung durch Erhitzen vervollständigt wird.

In der britischen Patentschrift Nr. 1 535 482 ist die Verwendung strahlungsempfindlicher Sulfoniumsalze von Arylsulfon-, Halogenarylsulfon-, Alkylsulfon- und Halogenalkylsulfonsäuren für die kationische Polymerisation von Epoxidharzen, Vinylmonomeren und -präpolymeren, cyclischen organischen Aethern, cyclischen organischen Estern, cyclischen organischen Sulfiden, cyclischen Aminen und cyclischen Organosiliciumverbindungen beschrieben.

U.S.Patentschrift Nr. 4 139 385 offenbart die Verwendung von Sulfoniumsalzen und weiteren Salzen bei der Härtung von Polyolefinen mittels Polythiolen in Gegenwart eines Oniumsalzkatalysators.

Aus der deutschen Offenlegungsschrift Nr. 2 833 648 ist es bekannt, dass man Triarylsulfoniumsalze unter Bestrahlung zur Auslösung der Härtung einer aliphatisch ungesättigten Verbindung, die eine 1,2-Epoxidgruppe enthält, wie Glycidylacrylat, oder eines Gemischs aus einem Epoxidharz mit einer aliphatisch ungesättigten Substanz wie Methylmethacrylat, einem Polyester oder Styrol verwenden kann.

In der U.S. Patentschrift Nr. 4 136 102 sind verschiedene ein Hexafluorophosphat-, Hexafluoroarsenat- oder Hexafluoroantimonatanion enthaltende Sulfoniumsalze sowie deren Verwendung bei der Härtung von Epoxidharzen beschrieben. Sie werden auch als nützlich zur Polymerisation einer Reihe nicht näher angegebener cyclischer organischer Verbindungen und cyclischer Organosiliciumverbindungen bezeichnet.

Aus der deutschen Offenlegungsschrift Nr. 2

730 725 ist die lichtinduzierte Härtung von ferner ein Polyvinylacetal enthaltenden Epoxidharzzusammensetzungen mittels aromatischer Oniumsalze bekannt.

In der U.S. Patentschrift Nr. 4 081 276 ist ein Verfahren zur Bildung von Photoresistabbildungen beschrieben, besonders zur Herstellung gedruckter Schaltungen, wobei eine Schicht eines Photoinitiators mit Strahlungsenergie belichtet und dann in Gegenwart eines Oniumsalzes mit einem kationisch polymerisierbaren Material, z.B. einem Epoxidharz, in Berührung gebracht wird.

In der belgischen Patentschrift Nr. 845 746 ist die Photopolymerisation von Gemischen aus einer Verbindung mit einer Epoxidfunktionalität vcn mehr als 1,5 Epoxidgruppen pro Molekül und einer Verbindung mit einer Hydroxylfunktionalität von mindestens eins unter Verwendung eines aromatischen Sulfoniumsalzes oder aromatischen Jodoniumsalzes als Katalysator beschrieben.

U.S. Patentschrift Nr. 4 090 936 beschreibt lichthärtbare flüssige Zusammensetzungen, die ein Oniumsalz als Photoinitiator enthalten.

U.S. Patentschrift Nr. 4 069 054 bezieht sich auf photopolymerisierbare Zusammensetzungen, die ein kationisch polymerisierbares Monomer, eine aromatische Sulfoniumverbindung und ein aromatisches tertiäres Amin, aromatisch tertiäres Diamin oder eine aromatische polycyclische Verbindung als Sensibilisator enthalten.

Ein aromatisches Sulfoniumsalz, nämlich Triphenylsulfonium-hexafluorophosphat, wurde bereits industriell zur Photopolymerisation von Epoxidharzen eingesetzt.

Es wurde nun überraschend gefunden, dass man kationisch polymerisierbare Materialien in Gegenwart gewisser aromatischer Sulfonylsulfoxoniumsalze als Katalysatoren mittels aktinischer Strahlung oder Hitze polymerisieren kann.

Im Gegensatz zu Sulfoniumsalze als Katalysatoren enthaltenden Zusammensetzungen des Standes der Technik setzen die erfindungsgemässen Zusammensetzungen bei der Bestrahlung keine übelriechenden Merkaptane frei.

Es wurde ferner gefunden, dass Harnstoff/Formaldehydharze, entgegen der Erwartung nach den Lehren der U.S. Patentschrift Nr. 4 102 687, bei Bestrahlung in Gegenwart eines erfindungsgemässen aromatischen Sulfonylsulfoxoniumsalzes gehärtet werden können, ohne dass Wärmezufuhr nötig wäre.

Gegenstand dieser Erfindung sind demnach aromatische Sulfonylsulfoxoniumsalze der Formel

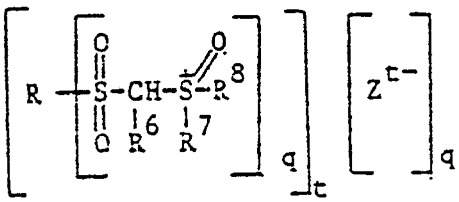

VI

worin q eine ganze Zahl von 1 bis 4 ist, R eine q-wertige aliphatische, cycloaliphatische oder aromatische Gruppe mit 1 bis 25 Kohlenstoffatomen bedeutet, die über eines ihrer Kohlenstoffatome direkt mit dem Schwefelatom der angegebenen benachbarten Sulfonylgruppe verknüpft ist, $R^6$ ein Wasserstoffatom, eine Alkyl- oder Aralkylgruppe mit 1 bis 25 Kohlenstoffatomen, eine Acylgruppe der Formel -$COR^9$ oder eine Gruppe der Formel

$$-CO-NH-(CO)_r-R^{10} \qquad VII$$

oder

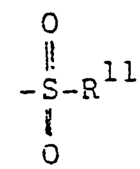

VIII

darstellt, wobei eines von R und $R^6$, aber nicht beide, für eine aromatische Gruppe mit 4 bis 25 Kohlenstoffatomen steht, $R^7$ eine Alkylgruppe mit 1 bis 12 Kohlenstoffatomen, eine Alkenylgruppe mit 2 bis 6 Kohlenstoffatomen, eine Cycloalkylgruppe mit 3 bis 8 Kohlenstoffatomen, eine Cycloalkylalkylgruppe mit 4 bis 10 Kohlenstoffatomen, eine Arylgruppe mit 4 bis 24 Kohlenstoffatomen oder eine Aralkylgruppe mit 5 bis 16 Kohlenstoffatomen bedeutet, $R^8$ dieselbe Bedeutung wie $R^7$ hat, aber auch für eine Dialkylaminogruppe mit 2 bis 6 Kohlenstoffatomen oder, falls $R^7$ Alkyl mit 1 bis 12 Kohlenstoffatomen darstellt, auch für eine Arylaminogruppe mit 4 bis 8 Kohlenstoffatomen stehen kann, $R^9$ einen Alkyl-Cycloalkyl-, Aryl- oder Aralkylrest mit 1 bis 25 Kohlenstoffatomen bedeutet, der über eines seiner Kohlenstoffatome direkt an die angegebene -CO- Gruppe gebunden ist, r null oder 1 ist, $R^{10}$ für einen Alkyl-, Cycloalkyl-, Aryl- oder Aralkylrest mit 1 bis 25 Kohlenstoffatomen steht, der über eines seiner Kohlenstoffatome bei r = null an das angegebene Stickstoffatom oder bei r = 1 an das Kohlenstoffatom der angegebenen benachbarten Carbonylgruppe direkt gebunden ist, $R^{11}$ einen über eines seiner Kohlenstoffatome direkt an das angegebene Schwefelatom gebundenen Alkyl-, Cycloalkyl-, Aryl- oder Aralkylrest mit 1 bis 25 Kohlenstoffatomen darstellt, wobei $R^9$, $R^{10}$ und $R^{11}$ eine aromatische Gruppe bedeuten, wenn R eine aliphatische oder cycloaliphatische Gruppe ist, t für 1, 2 oder 3 steht und $Z^{t-}$ das t-wertige Anion einer Protonensäure, vorzugsweise einer anorganischen Säure, bedeutet; wobei Dimethyl-p-toluolsulfonylmethylsulfoxoniumfluorid, Dimethyl-p-toluolsulfonylmethylsulfoxoniumchlorid, Dimethyl-p-methoxyphenylsulfonylmethyl sulfoxoniumfluorid, Dimethyl-p-methoxyphenyl sulfonylmethylsulfoxoniumchlorid, Dimethyl-benzylsulfonylmethylsulfoxonium fluorid und Dimethyl-benzylsulfonylmethylsulfoxoniumchlorid ausgeschlossen sind. Die Herstellung dieser nicht umfaßten Verbindungen wird von W. E. Truce und G. D. Madding in Tetrahedron Letters, 1966, 3681-3687 beschrieben.

Gegenstand dieser Erfindung sind weiterhin Zusammensetzungen, die aus (a) einer Verbindung bzw. einem Gemisch von Verbindungen, die unter dem Einfluss eines kationischen Katalysators in höhermolekulares Material überführbar sind, und (b) einem erfindungsgemässen aromatischen Sulfonylsulfoxoniumsalz bestehen. Dabei werden die oben spezifisch ausgeschlossenen Verbindungen der Formel VI von den erfindungsgemässen Zusammensetzungen umfasst.

Gegenstand dieser Erfindung ist ferner ein Verfahren zur Ueberführung einer Verbindung bzw. eines Gemischs von unter dem Einfluss eines kationischen Katalysators in höhermolekulares Material umwandelbaren Verbindungen in höhermolekulares Material, wobei man diese Verbindung bzw. dieses Gemisch in Gegenwart eines erfindungsgemässen aromatischen Sulfonylaulfoxoniumsalzes aktinischer Strahlung und/oder Hitze unterwirft.

In der Formel VI kann R beispielsweise einen aliphatischen Rest mit 1 bis 8 Kohlenstoffatomen und zwar einen Alkyl- oder Alkenylrest mit 1 bis 6 Kohlenstoffatomen, falls R einwertig ist, oder einen Alkylen- oder Alkenylenrest mit 1 bis 6 Kohlenstoffatomen, wenn R mehrwertig ist, bedeuten, wobei dieser Alkyl-, Alkenyl-, Alkylen- oder Alkenylenrest gegebenenfalls durch 1 bis 3 Chlor-, Fluor- oder Bromatome substituiert oder in der Kette durch ein Aethersauerstoffatom unterbrochen sein kann.

Andererseits kann R eine aromatische Gruppe mit 4 bis 25 Kohlenstoffatomen darstellen, die sowohl eine homocyclische als auch eine heterocyclische aromatische Gruppe sein kann. Unter "heterocyclische aromatische Gruppe" versteht man eine aromatische Gruppe, in der mindestens eine $-CH_2-$ oder $-CH=$ Gruppe des Rings einer aromatischen Verbindung durch ein von Kohlenstoff verschiedenes Atom, üblicherweise Stickstoff, Sauerstoff oder Schwefel, ersetzt ist. Beispiele für heterocyclisch aromatische Gruppen sind die 2-Furyl- und 2-Pyridylgruppen. Vorzugsweise bedeutet R eine homocyclische aromatische Gruppe mit 6 bis 25 Kohlenstoffatomen, beispielsweise eine tricyclische Gruppe wie eine Anthryl-, Phenanthryl- oder Fluorenylgruppe, bzw. eine Anthrylen-, Phenanthrylen- oder Fluorenylengruppe. Besonders bevorzugt steht R für

i) eine monocyclische oder dicyclische Aryl- oder Aralkylgruppe mit 6 bis 16 Kohlenstoffatomen, insbesondere eine Phenyl-, 2-Phenyläthyl-, Benzyl- oder Naphthylgruppe oder eine Gruppe der Formel

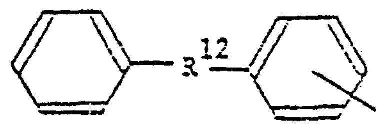

IX

worin $R^{12}$ eine Kohlenstoff-Kohlenstoffbindung, ein Aethersauerstoffatom oder eine Gruppe der Formel $-CH_2-$ oder $-C(CH_3)_2-$ bedeutet, oder

ii) eine monocyclische oder dicyclische Arylen- oder Aralkylengruppe mit 6 bis 16 Kohlenstoffatomen, insbesondere eine Phenylen-, Phenylenmethylen- ($-C_6H_4CH_2-$), Xylylen- oder Naphthylengruppe oder eine Gruppe der Formel

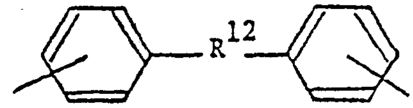

X

worin $R^{12}$ die unter der Formel IX angegebene Bedeutung hat, wobei diese Aryl-, Aralkyl-, Arylen- oder Aralkylengruppen gegebenenfalls in dem bzw. den aromatischen Ring(en) durch eins bis drei Chlor-, Fluor- oder Bromatome oder eins bis drei Alkyl- oder Alkoxygruppen mit jeweils 1 bis 4 Kohlenstoffatomen substituiert sind.

Spezielle Beispiele für geeignete Gruppen R sind Phenyl, p-Chlorphenyl, 3,4-Dichlorphenyl, o-Tolyl, p-Tolyl, p-Methoxyphenyl, 2,4-Toluylen, 2,6-Toluylen, Benzyl, 2-Phenyläthyl, o-, m- und p-Phenylen, p-Phenylenmethylen und Methylen-bis-(phenylen).

$R^7$ und $R^8$ stehen vorzugsweise je für eine Alkylgruppe mit 1 bis 4 Kohlenstoffstomen oder eine gegebenenfalls in dem bzw. den aromatischen Ring(en) durch eine oder zwei Alkyl- oder Alkoxygruppen mit jeweils 1 bis 4 Kohlenstoffatomen oder ein oder zwei Fluor-, Chlor- oder Bromatome substituierte Phenyl- oder Naphthylgruppe. Besonders bevorzugt stellen beide eine Methylgruppe dar.

Bedeutet R eine aromatische Gruppe, so können $R^9$, $R^{10}$ und $R^{11}$ jeweils eine aliphatische, cycloaliphatische oder aromatische Gruppe sein. Stellt R eine aliphatische oder cycloaliphatische Gruppe dar, so bedeuten $R^9$, $R^{10}$ und $R^{11}$ je eine aromatische Gruppe. Beispielsweise können $R^9$, $R^{10}$ und $R^{11}$ dieselbe Bedeutung wie R haben, wenn R eine einwertige Gruppe darstellt. Vorzugsweise steht R für eine aromatische Gruppe, und $R^9$ $R^{10}$ und $R^1$ bedeuten jeweils einen aliphatischen Rest mit 1 bis 8 Kohlenstoffatomen, insbesondere einen gegebenenfalls durch eins bis drei Chlor-, Fluor- oder Bromatome substituierten oder in der Kette durch ein Aethersauerstoffatom unterbrochenen Alkyl- oder Alkenylrest mit 1 bis 6 Kohlenstoffatomen.

Spezielle Beispiele für geeignete Gruppen $R^9$,

$R^{10}$ und $R^{11}$ sind Methyl-, Aethyl- und 2-Aethoxyäthylgruppen.

Wie oben erwähnt, muss, wenn R nicht für eine aromatische Gruppe steht, $R^6$ eine solche sein, wobei es dann eine Aralkylgruppe mit vorzugsweise 6 bis 16 Kohlenstoffatomen, einen Aracylrest mit vorzugsweise 6 bis 10 Kohlenstoffatomen, einen Aralkacylrest mit vorzugsweise 7 bis 17 Kohlenstoffatomen, eine Arylaminocarbonylgruppe mit vorzugsweise 6 bis 10 Kohlenstoffatomen, eine Aralkylaminocarbonylgruppe mit vorzugsweise 7 bis 11 Kohlenstoffatomen, eine Aracylaminocarbonylgruppe mit vorzugsweise 6 bis 16 Kohlenstoffatomen, eine Aralkacylaminocarbonylgruppe mit vorzugsweise 7 bis 17 Kohlenstoffatomen, eine Arylsulfonylgruppe mit vorzugsweise 6 bis 10 Kohlenstoffatomen oder eine Aralkylsulfonylgruppe mit vorzugsweise 7 bis 11 Kohlenstoffatomen darstellt.

$Z^{t-}$ ist das Anion einer Säure, die dazu fähig ist, die Polymerisation eines kationisch polymerisierbaren Materials zu bewirken. Es kann beispielsweise für $CH_3SO_4^-$ stehen, doch bedeutet es vorzugsweise $Cl^-$, $Br^-$, $NO_3^-$, $HSO_4^-$, $HSO_3^-$, $ClO_4^-$, $CF_3SO_3^-$, $CF_3COO^-$, $CH_3C_6H_4SO_3^-$, $H_2PO_4^-$, $SO_4^{--}$, $PO_4^{---}$ oder ein Anion der Formel

$MX_n^-$ XI

worin M ein Metall- oder Metalloidatom und X ein Halogenatom, vorzugsweise Fluor oder Chlor, bedeuten und n für 4, 5 und 6 und um eins grösser als die Wertigkeit von M ist, oder

$SbF_5(OH)^-$ XII.

M steht vorzugsweise für ein Bor- oder Wismut- und ganz insbesondere ein Antimon-, Arsen- oder Phosphoratom. Als Anion bzw. Anionen $MX_n^-$ kommen somit beispielsweise $BiCl_6^-$ oder $BF_4^-$ in Frage, doch steht $MX_n^-$ besonders bevorzugt für $PF_6^-$, $SbF_6^-$ oder $AsF_6^-$.

Die Sulfoxoniumsalze der Formel VI lassen sich wie folgt herstellen:

A. In der ersten Stufe setzt man ein Sulfonylfluorid der Formel

$R(SO_2F)_q$ XIII

mit 2q Molanteilen eines Oxosulfoniumylids der Formel

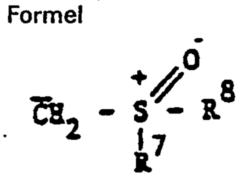

XIV

zu einem sulfonylhaltigen Ylid der Formel

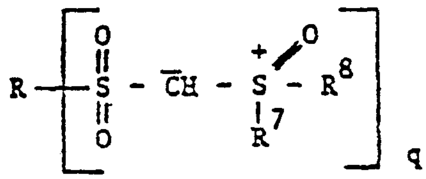

XV

um. Oxosulfoniumylide der Formel XIV sind aus Sulfoxoniumchloriden der Formel

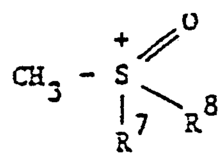

$Cl^-$ XVI

durch Behandlung mit starken Basen wie Natriumhydrid zugänglich (E.J. Corey und M. Chaykovsky, J. Amer. Soc., 1962, 84, 867). Die Reaktion solcher Ylide mit Alkyl-, Aryl- und Aralkylsulfonylfluoriden sowie die Herstellbarkeit einiger Dimethylarylsulfonylmethylsulfoxoniumchloride und -fluoride wird von W.E. Truce und G.D. Madding, Tetrahedron Letters, 1966, 3681 - 3687, beschrieben. Es werden jedoch keine Angaben über die Eigenschaften dieser Stoffe gemacht.

B. In der zweiten Stufe überführt man ein Ylid der Formel XV nach einer der folgenden Methoden in ein Sulfoxoniumsalz der Formel VI:

1. Sulfoxoniumsalze der Formel VI, worin $R^6$ für ein Wasserstoffatom steht, lassen sich durch Neutralisation eines Ylids der Formel XV mit $q/_t$ Molanteilen einer Protonensäure $H_tZ$, z.B. Salzsäure, Salpetersäure, Phosphorsäure, Tetrafluoroborsäure oder Hexafluorophosphorsäure erhalten.

2. Sulfoxoniumsalze der Formel VI, worin $R^6$ für eine Alkyl- oder Aralkylgruppe steht, lassen sich durch Umsetzung eines Ylids der Formel XV mit einem Alkylierungsmittel, beispielsweise einem Alkyl- oder Aralkylhalogenid, und nachfolgende Neutralisation mit $q/_t$ Molanteilen einer Protonensäure der Formel $H_tZ$ erhalten.

3. Sulfoxoniumsalze der Formel VI, worin $R^6$ für eine Gruppe der Formel $-COR^9$ steht, lassen sich durch Umsetzung eines Ylids der Formel XV mit einem Acylierungsmittel zwecks Einführung einer Gruppe der Formel $-COR^9$, z.B. einem Acylchlorid, und nachfolgende Neutralisation mit $q/_t$ Molanteilen einer Protonensäure der Formel $H_tZ$ erhalten.

4. Sulfoxoniumsalze der Formel VI, worin $R^6$ für eine Gruppe der Formel VII und r für null oder 1 stehen, lassen sich durch Umsetzung eines Ylids der Formel XV mit einem Isocyanat der Formel $R^{10}(CO)_rNCO$ und nachfolgende Neutralisation mit $q/_t$ Molanteilen einer Protonensäure der Formel $H_tZ$ erhalten.

5. Solche, worin $R^6$ für eine Gruppe der Formel VIII steht, lassen sich durch Umsetzung eines Ylids der Formel XV mit einem äquimolaren Anteil eines Sulfonylfluorids der Formel $R^{11}SO_2F$ zu einem Sulfonyloxosulfoniumylid der Formel XVII

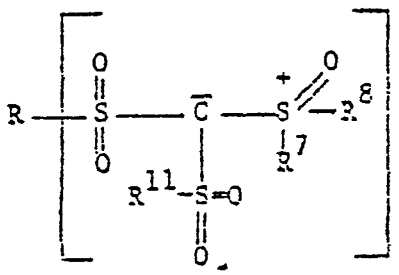

XVII

und nachfolgende Neutralisation mit $q/t$ Molanteilen einer Protonensäure der Formel $H_tZ$ erhalten.

Sulfoxoniumsalze der Formel VI, worin $R^6$ für eine Alkyl- oder Aralkylgruppe, eine Acylgruppe der Formel $-COR^9$ bzw. eine Gruppe der Formel VII steht, lassen sich aber auch durch Umsetzung eines Oxosulfoniumylids der Formel XIV mit einem Alkylierungsmittel, Acylierungsmittel bzw. Isocyanat, Umsetzung von 2q Molanteilen des entstandenen Ylids mit einem Sulfonylfluorid der Formel XIII nach der von Truce und Madding, a.a.O., beschriebenen Methode zu einem sulfonylhaltigen Ylid und dessen Neutralisation mit q/t Molanteilen einer Protonensäure der Formel $H_tZ$ herstellen.

Die Umsetzung von Oxosulfoniumyliden mit Alkylierungsmitteln, Acylierungsmitteln und Isocyanaten ist in der U.S. Patentschrift Nr. 3 442 901 beschrieben.

Wo eine bestimmte Protonensäure der Formel $H_tZ$ nicht zur Verfügung steht oder schwierig zu handhaben ist, kann man Salze wie Hexafluorophosphate und Hexafluoroantimonate durch doppelte Umsetzung mit den entsprechenden Chloriden oder anderen geeigneten Salzen herstellen. Beispielsweise kann man Dimethyltoluolsulfonylmethylsulfoxoniumhexafluorophosphat durch Ausfällen beim Zusatz einer wässrigen Lösung von Kaliumhexafluorophosphat zu einer wässrigen Lösung von Dimethyltoluolsulfonylmethylsulfoxoniumchlorid erhalten. Das entsprechende Hexafluoroantimonat kann durch Zugabe von festem Kaliumhexafluoroantimonat zur wässrigen Lösung des Chlorids hergestellt werden; löst man das Kaliumhexafluoroantimonat zunächst in Wasser, so ist das isolierte Produkt dann wegen Hydrolyse das Hydroxopentafluoroantimonat ($Z^{t-} = SbF_5(OH)^-$).

Spezielle Beispiele für geeignete Sulfonylsulfoxoniumsalze sind Dimethyl-p-toluolsulfonylmethylsulfoxoniumhexafluorophosphat sowie das entsprechende Hexafluoroantimonat, Dimethylphenylsulfonylmethylsulfoxoniumhexafluorophosphat und Tris-(dimethyl-p-toluolsulfonylmethylsulfoxonium)-orthophosphat.

Bei den erfindungsgemässen Zusammensetzungen wird eine genügende Menge (b) eingesetzt, um bei Belichtung der Zusammensetzung mit aktinischer Strahlung oder beim Erhitzen eine Polymerisation von (a) zu induzieren. Ueblicherweise setzt man 0,1 bis 7,5, insbesondere 0,5 bis 6, Gewichtsteile (b) auf 100 Gewichtsteile der Komponente (a) ein.

Die Komponente (a) kann beispielsweise ein Oxetan, ein Thi-iran oder ein Tetrahydrofuran sein. Vorzugsweise ist dies ein 1,2-Epoxid,ein Vinylmonomer oder -präpolymer, ein Aminoplast oder ein Phenoplast.

Im Fall eines 1,2-Epoxids muss $Z^{t-}$ in der Formel VI für $CF_3SO_3^-$, eine wie oben angegebene Gruppe der Formel $MX^-_n$ oder eine Gruppe der kormel $SbF_5OH^-$ stehen. Geeignete Mono-1,2-epoxide sind unter anderem Epichlorhydrin, Propylenoxyd, Glycidyläther eines einwertigen Alkohols oder Phenols, wie n-Butylglycidyläther oder Phenylglycidyläther, und Glycidylester, wie Glycidylacrylat oder -methacrylat.

Vorzugsweise ist es ein Epoxidharz, insbesondere ein solches, das mindestens eine direkt an ein Sauerstoffatom gebundene Gruppe der Formel

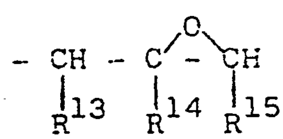

XVIII

enthält, worin entweder $R^{13}$ und $R^{15}$ je ein Wasserstoffatom darstellen, in welchem Fall $R^{14}$ ein Wasserstoffatom oder eine Methylgruppe bedeutet, oder $R^{13}$ und $R^{15}$ zusammen $-CH_2CH_2-$ darstellen, in welchem Fall $R^{14}$ ein Wasserstoffatom bedeutet.

Als Beispiele für solche Harze seien Polyglycidylund Poly-(β-methylglycidyl)-ester genannt, die man durch Umsetzung einer zwei oder mehr Carbonsäuregruppen pro Molekül enthaltenden Verbindung mit Epichlorhydrin, Glycerindichlorhydrin oder β-Methylepichlorhydrin in Gegenwart von Alkali erhalten kann. Solche Polyglycidylester können sich von aliphatischen Polycarbonsäuren, z.B. Bernsteinsäure, Glutarsäure, Adipinsäure, Pimelinsäure, Korksäure, Azelainsäure, Sebacinsäure oder dimerisierter oder trimerisierter Linolsäure, von cycloaliphatischen Polycarbonsäuren wie Tetrahydrophthalsäure, 4-Methyltetrahydrophthalsäure, Hexahydrophthalsäure und 4-Methyl-hexahydrophthalsäure und von aromatischen Polycarbonsäuren wie Phthalsäure, Isophthalsäure und Terephthalsäure ableiten. Weitere geeignete Glycidylester sind durch Vinylpolymerisation der Glycidylester von Vinylsäuren, insbesondere Glycidylacrylat und Glycidylmethacrylat, erhältlich.

Weitere Beispiele sind Polyglycidyl- und Poly-(β-

methylglycidyl)-äther, die durch Umsetzung einer mindestens zwei freie alkoholische und/oder phenolische Hydroxylgruppen pro Molekül enthaltenden Verbindung mit dem entsprechenden Epichlorhydrin unter alkalischen Bedingungen, oder auch in Gegenwart eines sauren Katalysators mit nachfolgender Alkalibehandlung erhältlich sind. Diese Aether lassen sich mit Poly-(epichlorhydrin) aus acyclischen Alkoholen wie Aethylenglykol, Diäthylenglykol und höheren Poly-(oxyäthylen)-glykolen, Propan-1,2-diol und Poly-(oxypropylen)-glykolen, Propan-1,3-diol, Poly-(oxytetramethylen)-glykolen, Pentan-1,5-diol, Hexan-2,4,6-triol, Glycerin, 1,1,1-Trimethylolpropan, Pentaerythrit und Sorbit, aus cycloaliphatischen Alkoholen wie Resorcit, Chinit, Bis-(4-hydroxycyclohexyl)-methan, 2,2-Bis-(4-hydroxycyclohexyl)-propan und 1,1-Bis-hydroxymethyl)-cyclohexen-3, sowie aus Alkoholen mit aromatischen Kernen, wie N,N-Bis-(2-hydroxyäthyl)-anilin und p,p'-Bis-(2-hydroxyäthylamino)-diphenylmethan, herstellen. Man kann sie ferner aus einkernigen Phenolen wie Resorcin und Hydrochinon, und mehrwertigen Phenolen wie Bis-(4-hydroxy-phenyl)-methan, 4,4'-Dihydroxydiphenyl, Bis-(4-hydroxy-phenyl)-sulfon, 1,1,2,2-Tetrakis-(4-hydroxyphenyl)-äthan, 2,2-Bis-(4-hydroxyphenyl)-propan (sonst als Bisphenol A bekannt) und 2,2-Bis-(3,5-dibrom-4-hydroxyphenyl)-propan, sowie aus Aldehyden wie Formaldehyd, Acetaldehyd, Chloral und Furfurol mit Phenol selbst und durch Chloratome oder Alkylgruppen mit jeweils bis zu neun Kohlenstoffatomen ringsubstituiertem Phenol, wie 4-Chlorphenol, 2-Methyl-phenol und 4-tert.-Butylphenol, gebildeten Novolaken herstellen.

Poly-(N-glycidyl)-verbindungen lassen sich ebenfalls verwenden, beispielsweise N-Glycidylderivate von Aminen wie Anilin, n-Butylamin, Bis-(4-aminophenyl)-methan und Bis-(4-methylaminophenyl)-methan, Triglycidylisocyanurat sowie N,N'-Diglycidylderivate von cyclischen Alkylenharnstoffen wie Aethylenharnstoff und 1,3-Propylenharnstoff, und Hydantoinen wie 5,5'-Dimethylhydantoin. Im allgemeinen sind diese jedoch nicht bevorzugt.

Ebenfalls kann man Poly-(S-glycidyl)-verbindungen einsetzen, z.B. Di-(S-glycidyl)-derivate von Dithiolen wie Aethan-1,2-dithiol und Bis-(4-mercaptomethylphenyl)-äther, doch werden auch diese nicht bevorzugt.

Beispiele für Epoxidharze mit Gruppen der Formel XVIII, worin $R^{13}$ und $R^{15}$ zusammen eine -$CH_2CH_2$-Gruppe bedeuten, sind Bis-(2,3-epoxycyclopentyl)-äther, 2,3-Epoxy-cyclopentylglycidyläther und 1,2-Bis-(2,3-epoxycyclo-pentyloxy)-äthan.

In Betracht kommen auch Epoxidharze, in denen die 1,2-Epoxidgruppen an Heteroatome verschiedener Art gebunden sind, z.B. der Glycidyläther-Glycidyl-ester der Salicylsäure.

Ebenfalls verwendbar sind Epoxidharze, in denen einige oder sämtliche Epoxidgruppen mittelständig sind, wie Vinylcyclohexendioxyd, Limonendioxyd, Dicyclopentadiendioxyd, 4-Oxatetracyclo [6.2.1.$0^{2,7}$.$0^{3,5}$]undecyl-9-glycidyläther, 1,2-Bis-(4-oxatetracyclo[6.2.1.$0^{2,7}$.$0^{3,5}$] un-decyl-9-oxy)-äthan, der 3,4-Epoxycyclohexylmethylester der 3',4'-Epoxycyclohexancarbonsäure sowie dessen 6,6'-Dime-thylderivat, der Bis-(3,4-epoxycyclohexancarbonsäureester) des Aethylenglykols, 3-(3,4-Epoxycyclohexyl)-8,9-epoxy-2,4-dioxaspiro[5,5]undecan sowie epoxidierte Butadiene oder Copolymere des Butadiens mit Aethylenverbindungen wie Styrol und Vinylacetat.

Gewünschtenfalls kann man Epoxidharzgemische verwenden.

Besonders bevorzugte, bei dem erfindungsgemässen Verfahren verwendete Epoxidharze sind gegebenenfalls vorverlängerte Diglycidyläther von zweiwertigen Phenolen, wie 2,2:Bis-(4-hydroxyphenyl)-propan und Bis-(4-hydroxyphenyl)-methan, und von zweiwertigen aliphatischen Alkoholen wie Butan-1,4-diol.

Gewünschtenfalls kann man das Epoxidharz einer Mischhärtung mit einem mehrwertigen Alkohol, d.h. einer Verbindung mit mindestens zwei alkoholischen, vorzugsweise primären Hydroxylgruppen im Molekül, unterwerfen. Vorzugsweise liegt der mehrwertige Alkohol in genügender Menge vor, um 0,5 bis 1,5, insbesondere 0,75 bis 1,25, alkoholische Hydroxylgruppen pro 1,2-Epoxidgruppe des Epoxidharzes zu liefern. Der mehrwertige Alkohol enthält vorzugsweise ausser den alkoholischen Hydroxylgruppen nur Kohlenstoff-, Wasserstoff- und gegebenenfalls als Aethersauerstoff oder Acetal- oder Carbonylgruppen vorhandene Sauerstoff- sowie Halogenatome. Ferner besitzt der mehrwertige Alkohol vorzugsweise ein Molekulargewicht von mindestens 100 und insbesondere höher als 1000. Geeignete mehrwertige Alkohole sind beispielsweise Poly-(oxyäthylen)glykole, Poly-(oxypropylen)-glykole, Poly-(oxytetramethylen)-glykole, Polyepichlorhydrine, Poly-(oxyäthylen)-, Poly-(oxypropylen)- bzw. Poly-(oxytetramethylen)-triole, die durch Polymerisation von Aethylenoxyd, Propylenoxyd bzw. Tetrahydrofuran in Gegenwart von Glycerin oder 1,1,1-Trimethylolpropan erhältlich sind, Polycaprolactone mit Hydroxylendgruppen, Copolymere des Styrols mit Allylalkohol, Polyvinylalkohole, Hydroxypropylcellulose, hydroxylhaltige Polyvinylacetale und Teilester der Cellulose, z.B. ein Celluloseacetatbutyrat.

Vinylmonomere und -präpolymere, die dabei polymerisiert werden können, sind unter anderem Styrol, α-Methylstyrol, Allylbenzol, Divinylbenzol, Vinylcyclohexan, 4-Vinylcyclohexen-1, N-Vinylpyrrolidinon-2, N-Vinylcarbazol, Acrolein, Isopren, Butadien, Piperylen, Vinylacetat und Vinyläther wie Isobutylvinyläther, Methylvinyläther, Trimethylolpropan trivinyläther, Glycerintrivinyläther, die Vinyläther von Aethylenglykol und Poly-(oxyäthylenglykolen) und

cyclische Vinyläther mit mindestens zwei cyclischen Vinyläthergruppen, die jeweils einen Teil eines 3,4-Dihydro-2H-pyrankerns bilden, wie der 3,4-Dihydro-2H-pyranyl-(2)-methylester der 3,4-Dihydro-2H-pyran-2-carbonsäure und dessen Präpolymere. Die bevorzugten Vinylverbindungen sind Vinyläther von aliphatischen einwertigen Alkoholen sowie der 3,4-Dihydro-2H-pyranyl-(2)-methylester der 3,4-Dihydro-2H-pyran-2-carbonsäure und dessen Präpolymere.

Die als Komponente (a) bevorzugten Aminoplaste enthalten pro Molekül mindestens zwei direkt an ein Amid- oder Thioamidstickstoffatom bzw. -atome gebundene Gruppen der Formel $-CH_2OR^{16}$, worin $R^{16}$ ein Wasserstoffatom, eine Alkylgruppe mit 1 bis 4 Kohlenstoffatomen oder eine Acetylgruppe bedeutet. Beispiele für solche Aminoplaste sind die N-Hydroxymethyl-, N-Methoxymethyl-, N-Butoxymethylund N-Acetoxymethylderivate der folgenden Amide und amidartigen Substanzen:

I. Harnstoff, Thioharnstoff und cyclische Harnstoffe der Formel

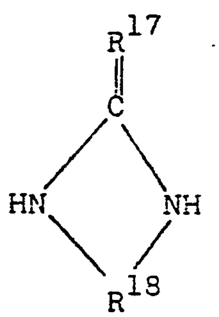

XIX

worin $R^{17}$ Sauerstoff oder Schwefel und $R^{18}$ entweder eine Gruppe der Formel

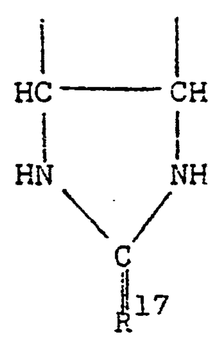

XX

oder eine gegebenenfalls durch Methyl-, Methoxy- oder Hydroxylgruppen substituierte und gegebenenfalls durch -CO-, -O- oder $-N(R^{19})-$, wobei $R^{19}$ für eine Alkyl- oder Hydroxyalkylgruppe mit bis zu 4 Kohlenstoffatomen steht, unterbrochene zweiwertige Gruppe mit 2 bis 4 Kohlenstoffatomen darstellen.

Solche cyclische Harnstoffe sind beispielsweise Aethylenharnstoff (Imidazolidinon-2), Dihydroxyäthylenharnstoff (4,5-Dihydroxyimidazolidinon-2), Hydantoin, Uron (Tetrahydro-oxadiazinon-4), 1,2-Propylenharnstoff, (4-Methylimidazolidinon-4), 1,3-Propylenharnstoff, (Hexa-hydro-2H-pyrimidon-2), Hydroxypropylenharnstoff (5-Hydroxy-hexahydro-2H-pyrimidon-2), Dimethylpropylenharnstoff (5,5-Dimethylhexahydro-2H-pyrimidon-2), Dimethylhydroxypropylenharnstoff bzw. Dimethylmethoxypropylenharnstoff (4-Hydroxy-bzw. 4-Methoxy-5,5-dimethylhexahydro-2H-pyrimidon-2), 5-Aethyltriazinon-2 und 5-(2-Hydroxyäthyl)-triazinon-2.

II. Carbamate and Dicarbamate aliphatischer einwertiger und zweiwertiger Alkohole mit bis zu vier Kohlenstoffatomen, z.B. Methyl-, Aethyl-, Isopropyl-, 2-Hydroxyäthyl-, 2-Methoxyäthyl-, 2-Hydroxy-n-propyl- und 3-Hydroxy-n-propylcarbamat sowie Aethylen- und 1,4-Butylendicarbamate.

III. Melamin und weitere Polyamino-1,3-triazine wie Acetoguanamin, Benzoguanamin und Adipoguanamin.

Gewünschtenfalls kann man Aminoplaste einsetzen, die sowohl N-Hydroxymethyl- als auch N-Alkoxymethyl- oder N-Hydroxymethyl- und N-Acetoxymethylgruppen enthalten (beispielsweise ein Hexamethylolmelamin, in dem 1 bis 3 Hvdroxylgruppen mit Methylgruppen veräthert sind).

Die bevorzugten Aminoplaste sind Kondensationsprodukte des Harnstoffs, Urons, Hydantoins oder Melamins mit Formaldehyd sowie teilweise oder völlig verätherte Produkte solcher Kondensationsprodukte mit einem aliphatischen einwertigen Alkohol mit 1 bis 4 Kohlenstoffatomen.

Als Phenoplaste werden aus einem Phenol und einem Aldehyd hergestellte Resole bevorzugt. Als Phenole eignen sich unter anderem Phenol selbst, Resorcin, 2,2-Bis-(p-hydroxyphenyl)-propan, p-Chlorphenol, ein durch ein oder zwei Alkylgruppen mit jeweils 1 bis 9 Kohlenstoffatomen substituiertes Phenol, wie o-, m- und p-Kresol, die Xylenole, p-tertiär-Butylphenol, p-Nonylphenol und phenylsubstituierte Phenole, insbesondere p-Phenylphenol. Der mit dem Phenol kondensierte Aldehyd ist vorzugsweise Formaldehyd, doch kommen auch andere Aldehyde wie Acetaldehyd und Furfurol in Frage. Gewünschtenfalls kann man ein Gemisch aus solchen härtbaren Phenol/Aldehydharzen verwenden.

Die bevorzugten Resole sind Kondensationsprodukte des Phenols, p-Chlorphenols, Resorcins oder o-, m- oder p-Kresols mit Formaldehyd.

Vorzugsweise enthalten die erfindungsgemässen Zusammensetzungen, wenn sie photopolymerisiert werden sollen, auch einen Sensibilisator. Es wurde gefunden, dass die Einarbeitung geeigneter Sensibilisatoren die Härtungsgeschwindigkeit noch weiter steigert, was die Anwendung noch kürzerer Belichtungszeiten und/oder weniger starker Bestrahlungsquellen ermöglicht. Ausserdem wird die Empfindlichkeit für sichtbares Licht erhöht.

Von Farbstoffen verschiedene Sensibilisatoren haben sich als wirksamer erwiesen, insbesondere aromatische polycyclische Verbindungen mit mindestens drei kondensierten Benzolringen und mit einer Ionisationsenergie unter etwa 7,5 eV. Geeignete Sensibilisatoren dieser Art sind in der U.S. Patentschrift Nr. 4 069 054 beschrieben und schliessen Anthracen, Rubren, Perylen, Phenanthren, Fluoranthen und Pyren ein. Vorzugsweise arbeitet man 0,1 bis 2% und insbesondere 0,25 bis 0,75 Gew.% des Sensibilisators, berechnet auf das Gewicht von (a), ein.

Beim Photopolymerisationsvorgang verwendet man vorzugsweise aktinische Strahlung einer Wellenlänge von 200 bis 600 nm. Als aktinische Strahlungsquellen eignen sich unter anderem Kohlelichtbögen, Quecksilberdampflichtbögen, Leuchtröhren mit ultraviolettes Licht ausstrahlenden Leuchtstoffen, Argon- und Xenonglimmlampen, Wolframlampen und photographische Flutlampen. Darunter sind Quecksilberdampflichtbögen, insbesondere Höhensonnen, fluoreszierende Höhensonnen und Metallhalogenidlampen am besten geeignet. Die zur Belichtung erforderliche Zeit wird von verschiedenen Faktoren abhängen, unter anderem beispielsweise dem jeweils verwendeten polymerisierbaren Substrat, der Art der Lichtquelle und deren Abstand vom bestrahlten Material. Der mit Photopolymerisationsmethoden vertraute Fachmann kann die geeigneten Zeiten leicht bestimmen. Wenn es wie in dem unten beschriebenen Verfahren erforderlich ist, dass das so photopolymerisierte Produkt beim Erhitzen mit einem eingemischten Heisshärter noch härtbar ist, dann muss die Bestrahlung natürlich bei einer Temperatur unterhalb derjenigen, bei der weitgehende Heisshärtung des photopolymerisierten Produkts durch den Heisshärter eintreten würde, erfolgen.

Wenn die erfindungsgemässen Zusammensetzungen im wesentlichen mittels Hitze allein polymerisiert werden sollen, so erhitzt man sie vorzugsweise auf eine Temperatur von 100°C bis 175°C, vorzugsweise für 3 bis 30 Minuten.

Die erfindungsgemässen Zusammensetzungen sind als Oberflächenbeschichtungen verwendbar. Man kann sie, vorzugsweise als Flüssigkeit, auf ein Substrat wie Stahl, Aluminium, Kupfer, Cadmium, Zink, Papier oder Holz aufbringen und bestrahlen oder erhitzen. Photopolymerisiert man einen Teil der Beschichtung, wie bei der Bestrahlung durch eine Maske hindurch, so kann man die unbelichteten Stellen mit einem Lösungsmittel auswaschen, um unpolymerisierte Anteile zu entfernen, während die photopolymerisierten, unlöslichen Anteile an Ort und Stelle verbleiben. Die erfindungsgemässen Zusammensetzungen sind somit bei der Herstellung von Druckplatten und gedruckten Schaltungen verwendbar. Methoden zur Herstellung von Druckplatten und gedruckten Schaltungen aus photopolymerisierbaren Zusammensetzungen sind wohlbekannt (siehe z.B. britische Patentschrift Nr. 1 495 746).

Die Zusammensetzungen lassen sich auch als Klebstoffe verwenden. Eine Schicht der Zusammensetzung kann zwischen zwei Oberflächen von Gegenständen eingebracht werden, und der Aufbau wird dann erhitzt bzw. bestrahlt und gewünschtenfalls erhitzt, um die Polymerisation zu vervollständigen. Wenn photopolymerisiert werden soll, ist es natürlich erforderlich, dass mindestens einer der Gegenstände für aktinische Strahlung durchlässig ist, d.h. beispielsweise aus Glas besteht.

Die Zusammensetzungen sind ferner bei der Herstellung von faserverstärkten Verbundstoffen, einschliesslich Plattenpressmassen, verwendbar.

Dabei kann man sie in flüssiger Form, kontinuierlich oder diskontinuierlich direkt auf Verstärkungsfasern (einschliesslich Spinnfäden, Endlosfäden oder Whiskers) auftragen, die in Form von Gewebe oder Faservlies, kettenstarken Schnitten oder Stapelseide vorliegen, insbesondere von Fasern aus Glas, Bor, Edelstahl, Wolfram, Aluminiumoxyd, Siliciumcarbid, Asbest, Kaliumtitanatwhiskers, einem aromatischen Polyamid wie Poly-(m-phenylenisophthalamid), Poly-(p-phenylenterephthalamid) oder Poly-(p-benzamid), Polyäthylen, Polypropylen oder Kohlenstoff.

Der faserverstärkte Verbundstoff kann auch nach einem diskontinuierlichen Verfahren oder kontinuierlich aus Folien der photopolymerisierten Zusammensetzung hergestellt werden. Beim diskontinuierlichen Verfahren wird das faserförmige Verstärkungsmittel auf eine Folie aus photopolymerisierter Zusammensetzung, die zweckmässigerweise etwas unter Spannung steht, aufgelegt, und wenn erwünscht, eine zweite solche Folie darübergelegt, und der Aufbau wird dann unter Erhitzen verpresst. Auch ist kontinuierliche Herstellung dadurch möglich, dass man das faserförmige Verstärkungsmaterial mit der Folie aus photopolymerisierter Zusammensetzung in Berührung bringt und dann gewünschtenfalls eine zweite solche Folie auf die Rückseite des faserförmigen Verstärkungsmaterials legt und Hitze und Druck zur Einwirkung bringt. Zweckmässiger bringt man zwei solche, vorzugsweise auf der Rückseite durch Bänder oder abziehbare Blätter abgestützte Folien gleichzeitig auf das faserförmige Verstärkungsmittel so auf, dass die beiden freiliegenden Seiten in Berührung kommen. Werden zwei solche Folien aufgebracht, so können diese gleich oder verschieden sein.

Vielschichtige Verbundstoffe lassen sich dadurch herstellen, dass man abwechselnde Folien und Schichten aus einem oder mehreren faserförmigen Verstärkungsmaterialien unter Druck erhitzt. Bei Verwendung kettenstarker Fasern als Verstärkungsmittel können deren aufeinanderfolgende Schichten so orientiert sein, dass sich gekreuzte Lagen ergeben.

Gegebenenfalls kann man mit dem

faserförmigen Verstärkungsmaterial zusätzliche Verstärkungstypen wie eine Metallfolie (z.B. aus Aluminium, Stahl oder Titan) oder eine Kunststofffolie (z.B. aus einem aromatischen oder aliphatischen Polyamid, Polyimid, Polysulfon oder Polycarbonat) oder Kautschukfolie (z.B. aus Neopren oder Acrylnitrilkautschuk) verwenden.

Man kann aber auch ein Gemisch aus den Verstärkungsfasern und einer erfindungsgemässen Zusammensetzung direkt unter Bildung eines Verbundstoffs erhitzen.

Bei der Herstellung von Plattenpressmassen wird eine erfindungsgemässe Zusammensetzung mit dem Stapelseideverstärkungsmaterial und gegebenenfalls weiteren Komponenten schichtweise einer Bestrahlung durch Trägerfolien hindurch ausgesetzt bzw. erhitzt.

Soviel polymerisierbare Zusammensetzung wird vorzugsweise eingesetzt, dass der Verbundstoff insgesamt 20 bis 80 Gew.% dieser Zusammensetzung und dementsprechend 80 bis 20 Gew.% Verstärkung enthält. Besonders bevorzugt verwendet man 30 bis 50 Gew.% der Zusammensetzung.

Die erfindungsgemässen Zusammensetzungen sind zur Herstellung von Kitten und Spachtelmassen verwendbar. Sie lassen sich als Tauchbeschichtungen anwenden, wobei ein zu beschichtender Artikel in die flüssige Zusammensetzung getaucht und wieder entnommen und die anhaftende Beschichtung zur Photopolymerisation (und damit Verfestigung) erhitzt bzw. bestrahlt und gewünschtenfalls anschliessend erhitzt wird.

Es wurde gefunden, dass es möglich ist, unter Verwendung der erfindungsgemässen Salze der Formel VI Epoxidharze und Phenoplaste in zwei Stufen zu härten; zunächst wird das Harz durch Belichtung mit aktinischer Strahlung in Gegenwart des Sulfoxoniumsalzes und eines latenten, hitzeaktivierbaren Vernetzungsmittels für das Epoxidharz oder Phenoplast in die teilweise gehärtete B-Stufe umgewandelt, und in einer zweiten Stufe wird die teilweise gehärtete Zusammensetzung so erhitzt, dass die Härtung mittels des hitzeaktivierbaren Vernetzungsmittels zuendegeht. So kann man eine flüssige oder halbflüssige Zusammensetzung herstellen, die dann geformt oder zur Imprägnierung eines Substrats verwendet werden kann, während sie zur Verfestigung bestrahlt wird; der verfestigte Körper wird dann zur gewünschten Zeit erhitzt, um die Härtung des Harzes zu vervollständigen.

Gemäss einer weiteren Ausführungsform dieser Erfindung wird daher ein Epoxidharz oder ein Phenoplast in Gegenwart einer zur Polymerisation des Epoxidharzes oder Phenoplastes wirksamen Menge eines Sulfoxoniumsalzes der Formel VI und einer härtenden Menge eines latenten Heisshärters für das Epoxidharz oder Phenoplast unter Bildung des B-Stufenprodukts bestrahlt, und die Härtung der Zusammensetzung wird zur gewünschten Zeit durch Erhitzen vervollständigt.

Eine weitere Ausführungsform umfasst eine Zusammensetzung, die ein Epoxidharz oder Phenoplast, eine zur Polymerisation dieses Epoxidharzes oder Phenoplastes bei Belichtung der Zusammensetzung mit aktinischer Strahlung wirksame Menge eines Sulfoxoniumsalzes der Formel VI und eine härtende Menge eines latenten Heisshärters für das Epoxidharz oder Phenoplast enthält.

Als hitzeaktivierbare Vernetzungsmittel für die Epoxidharzzusammensetzungen eignen sich unter anderem Polycarbonsäureanhydride, Komplexe von Aminen, insbesondere primären oder tertiären aliphatischen Aminen wie Aethylamin, Trimethylamin und n-Octyldimethylamin, mit Bortrifluorid oder Bortrichlorid, und latente Bordifluoridchelate. Aromatische Polyamine und Imidazole sind im allgemeinen nicht bevorzugt, da sie ziemlich schlechte Resultate geben, möglicherweise wegen einer Reaktion zwischen dem freigesetzten sauren Katalysator und dem Amin. Dicyandiamid kann mit Erfolg verwendet werden, solange es in Form relativ grober Teilchen vorliegt.

Geeignete hitzeaktivierbare Vernetzungsmittel für Resole sind unter anderem Hexamethylentetramin und Paraformaldehyd.

Die für die Heisshärtung nach der Photopolymerisation erforderliche Temperatur und Erhitzungsdauer sowie die Anteile an hitzeaktivierbarem Härter werden leicht durch Reihenversuche ermittelt und lassen sich ohne weiteres aus dem wohlbekannten Fachwissen über die Heisshärtung von Epoxidharzen und Phenol/Aldehydresolen ableiten.

Zusammensetzungen, welche Harze mit Epoxidgruppen oder phenolischen Hydroxylgruppen aufweisen, über die sie nach der Photopolymerisation heissgehärtet werden können, sind bei der Herstellung von mehrlagigen gedruckten Schaltungen besonders nützlich.

Herkömmlicherweise wird eine mehrlagige gedruckte Schaltung aus mehreren zweiseitigen Kupferleiterplatten hergestellt, die übereinander gestapelt und voneinander durch Isolierbögen, üblicherweise aus mit einem Epoxidharz oder Phenol/Formaldehydharz in der B-Stufe imprägnierter Glasfaser, getrennt werden. Wird kein Heisshärter in die photopolymerisierbare Harzschicht in der Leiterplatte eingemischt, so kann man sie in die mit den Platten alternierenden Isolierschichten einbringen, welche zweckmässig aus einem Epoxidharz- oder Phenol/Formaldehydharzprepreg bestehen; vorausgesetzt der Pretreg ist nicht zu dick, wandert genügend darin enthaltener Heisshärter aus, um die Vernetzung des photopolymerisierten Epoxidharzes oder Phenol/Formaldehydharzes auszulösen. Zur gegenseitigen Verklebung der Schichten wird der Stapel erhitzt und verpresst. Herkömmliche photopolymerisierbare Materialien bilden jedoch weder mit Kupfer noch mit harzimprägnierten Glasfaserfolien einen starken Verbund. Wird ein Stapel verklebt, während das Photopolymer noch das Kupfer überdeckt, so ist er deshalb an sieh schwaeh und kann im

Gebrauch auseinanderblättern. In der normalen Praxis entfernt man deshalb das restliehe Photopolymer nach der Aetzstufe entweder mittels starker Lösungsmittel oder durch eine mechanische Methode, z.B. mit Bürsten. Ein solehes Abstreifverfahren kann das Kupfer der gedruekten Schaltung oder die Oberfläehe des Verbunds, auf dem die Schaltung liegt, beschädigen, und es besteht daher ein Bedarf für eine Methode, bei der es nicht mehr nötig wäre, das photopolymerisierte Material vor dem gegenseitigen Verkleben der Platten zu entfernen. Die Gegenwart verbleibender Vernetzungsgruppen in den erfindungsgemässen Zusammensetzungen hat zur Folge, dass bei der Verklebung der Platten eine Vernetzung eintreten kann, was zu guter Haftung am Kupfer und am harzimprägnierten Glasfasersubstrat führt, sodass der eben erwähnte Schritt entfällt; auch erhält man dabei Produkte mit einer höheren Glasübergangstemperatur.

Eine weitere Anwendung unter Heisshärtung nach der Photopolymerisation der erfindungsgemässen Zusammensetzungen betrifft das Filamentwickeln. Dabei wird ein endloses Kabel der faserförmigen Verstärkung mit einer einen latenten Heisshärter enthaltenden Zusammensetzung imprägniert und dann unter gleichzeitiger Belichtung der Wicklung mit aktinischer Strahlung um einen Dorn oder Spulkörper gewickelt. Derartige Filamentwicklungen besitzen noch einen gewissen Grad Biegsamkeit, was gestattet, den Dorn oder Spulkörper leichter zu entfernen, als es bei einer in einem Schritt gebildeten starren Wicklung möglich wäre. Erforderlichenfalls kann man die Wicklung zur Vernetzung der Zusammensetzung erhitzen.

Bei einer weiteren solchen Anwendung wird eine Schicht der Zusammensetzung in flüssiger Form bis zur Verfestigung bestrahlt, wobei eine Klebfolie entsteht, die dann zwischen und in Berührung mit zwei zu verklebenden Oberflächen eingebracht wird, und der Aufbau wird erhitzt, um die Vernetzung der Zusammensetzung zu vervollständigen. Auf einer Seite kann die Folie mit einem abziehbaren Unterlegblatt, z.B. aus einem Polyolefin oder Polyester oder einem cellulosehaltigen Papier mit einer Silikonbeschichtung als Trennmittel, versehen sein. Häufig lässt sich der Aufbau leichter handhaben, wenn die Folie eine klebrige Oberfläche aufweist. Dies lässt sich dadurch erzielen, dass man die Folie mit einer Substanz beschichtet, die bei Raumtemperatur klebrig ist, aber unter den zur Vervollständigung der Vernetzung der Zusammensetzung angewandten Hitzebedingungen zu einem harten, unlöslichen, unschmelzbaren Harz vernetzt. Jedoch liegt ein ausreichender Klebrigkeitsgrad häufig ohne zusätzliche Behandlung vor, insbesondere wenn die Polymerisation der Zusammensetzung nicht zu weit fortgeschritten ist. Geeignete Klebsubstrate schliessen Metalle wie Eisen, Zink, Kupfer, Nickel und Aluminium, Keramik, Glas und Kautschuktypen ein.

Die nachfolgenden Beispiele erläutern die Erfindung. Teile sind Gewichtsteile, falls nicht anders angegeben.

Die Beispiele 1 bis 4 erläutern die Herstellung von erfindungsgemässen Sulfoxoniumsalzen.

## BEISPIEL 1

### Herstellung von Dimethyl-p-toluolsulfonylmethylsulfoxoniumhexafluorophosphat

Nach der von Corey und Chaykovsky, a.a.O., beschriebenen Methode stellt man Dimethylsulfoxoniummethylid aus Trimethylsulfoxoniumchlorid (25,7 Teile) durch Behandlung mit Natriumhydrid (6,7 Teile) in trockenem Tetrahydrofuran her. Dieses Ylid wird durch Umsetzung mit p-Toluolsulfonylfluorid (17,4 Teile) nach der von Truce und Madding, a.a.O., beschriebenen Methode in Dimethylsulfoxonium-p-toluolsulfonylmethylid überführt. Das Toluolsulfonylmethylid neutralisiert man im Verlauf von 15 Minuten durch Zugabe von 65%iger wässriger Hexafluorophosphorsäure (49,5 Teile) und engt die Lösung ein. Der ausgefallene weisse Feststoff wird abfiltriert, mit Wasser gewaschen und im Vakuum getrocknet, wobei man 20 Teile des gewünschten Produkts erhält (VI, R = p-$CH_3C_6H_4$, $R^6$ = H, $R^7$ = $R^8$ = $CH_3$, q = t = 1, $Z^{t-}$ = $PF_6^-$), Schmelzpunkt 174-5°C; NMR (Aceton-$d_6$) 2,50 (s-3H), 4,23 (s-6H), 6,20 (s-2H) und 7,5-8,2 (m-4H); IR (KBr-Scheibe) 3020, 3000, 2930, 2920, 1590, 1400, 1330, 1300, 1250, 1160, 1030, 940, 840 und 750 $cm^{-1}$; UV (Aethanol)$\lambda_{max}$ 255 nm (die Daten gelten für aus Methanol umkristallisiertes Produkt).

## BEISPIEL 2

### Herstellung von Dimethylphenylsulfonylmethylsulfoxoniumhexafluorophosphat

Dimethylsulfoxoniummethylid wird wie im vorhergehenden Beispiel beschrieben hergestellt. Dieses Ylid wird durch Umsetzung mit Benzolsulfonylfluorid (20 Teile) nach der von Truce und Madding, a.a.O., beschriebenen Methode in Dimethylsulfoxoniumbenzolsulfonylmethylid überführt. Das Benzolsulfonylmethylid neutralisiert man durch Zugabe von 65%iger wässriger Hexafluorophosphorsäure (49,5 Teile) und engt dann die Lösung ein. Der ausgefallene weisse Feststoff wird abfiltriert, mit Wasser gewaschen und im Vakuum getrocknet, wobei man 23 Teile des gewünschten Produkts erhält (VI, R = $C_6H_5$, $R^6$ = H, $R^7$ = $R^8$ = $CH_3$, t = q = 1 $Z^{t-}$ = $PF_6^-$), Schmelzpunkt 135-7°C; NMR (Aceton-$d_6$) 4,23 (s-

6H), 6,0 (s-2H) und 7,7-8,2 (m-5H); IR (KBr-Scheibe) 3020, 3000, 2930, 1610, 1450, 1350, 1330, 1240, 1160, 1080, 1030, 840 und 750 $cm^{-1}$; UV (Aethanol) $\lambda_{max}$ 257 nm (die Daten gelten für aus Methanol umkristallisiertes Produkt).

**BEISPIEL 3**

**Herstellung von Dimethyl-p-toluolsulfonylmethylsulfoxoniumhexafluoroantimonat**

Einen weiteren Anteil des zuvor hergestellten Toluolsulfonylmethylids (2,46 g) löst man in 20 ml 0,5-m-Salzsäure, gibt unter Rühren Natriumhexafluoroantimonat (2,6 g) dazu und rührt noch weitere 30 Minuten. Das gewünschte Hexafluoroantimonat (VI, R = p-$CH_3C_6H_4$, $R^6$ = H, $R^7$ = $R^8$ = $CH_3$, q = t = 1, $Z^{t-}$ = $SbF_6$) wird abfiltriert und im Vakuum getrocknet. Ausbeute: 1,9 g.

**BEISPIEL 4**

**Herstellung von Tris-(dimethyl-p-toluolsulfonylmethylsulfoxonium)-orthophosphat**

Die Lösung von weiteren 2,46 g des Toluolsulfonylmethylids in 20 ml 0,5-m-Salzsäure versetzt man unter Rühren mit 1,40 g Silberphosphat, filtriert das ausgefallene Silberchlorid ab und lässt das Filtrat über Nacht stehen. Das gewünschte Phosphat (VI, R = p-$CH_3C_6H_4$, $R^6$ = H, $R^7$ = $R^8$ = $CH_3$, q = 1, t = 3, $Z^{t-}$ = $PO_4^{---}$) scheidet sich als farblose Kristalle aus.

**BEISPIEL 5**

Je 96 Teile 2,2-Bis-(p-glycidyloxyphenyl)-propan und entweder 4 Teile Dimethyl-p-toluolsulfonylmethylsulfoxoniumhexafluorophosphat oder 4 Teile des entsprechenden Hexafluoroantimonats enthaltende Gemische bringt man als 10 µm dicke Filme auf Weissblech auf. Die Filme werden im Abstand von 8 cm mit der Strahlung einer Mitteldruckquecksilberbogenlampe (80 W per cm) belichtet. Dabei erhält man jeweils nach 10 Sekunden einen klebfreien, lösungsmittelfesten Ueberzug.

**BEISPIEL 6**

Eine aus 98,7 Teilen 2,2-Bis-(p-glycidyloxy-phenyl)-propan und 1,3 Teilen Dimethyl-p-toluolsulfonylmethylsulfoxoniumhexafluorophosphat bestehende Zusammensetzung bringt man als 10 µm dicken Film auf Weissblech auf und erhitzt 20 Minuten auf 150°C ohne Strahlungsbelichtung. Dabei erhält man einen harten, klebfreien Ueberzug.

**BEISPIEL 7**

Ein Gemisch aus 96 Teilen des 3,4-Epoxycyclohexylesters der 3,4-Epoxycyclohexancarbonsäure und 4 Teilen Dimethyl-p-toluolsulfonylmethylsulfoxoniumhexafluorophosphat bringt man als 10 µm dicken Film auf Weissblech auf. 5 Sekunden Bestrahlung des Films unter den in Beispiel 5 beschriebenen Bedingungen liefert einen klebfreien Ueberzug.

**BEISPIEL 8**

Eine aus 96 Teilen eines handelsüblichen Phenol/ Formaldehydresols mit einem P:F-Molverhältnis von 1:1,6 und 4 Teilen Dimethyl-p-toluolsulfonylmethylsulfoxoniumhexafluorophosphat bestehende Zusammensetzung bringt man als 10 µm dicken Film auf Weissblechaufundbestrahlt wie in Beispiel 5 beschrieben. Dabei erhält man nach 10 Sekunden einen klebfreien Ueberzug.

**BEISPIEL 9**

Man verfährt wie in Beispiel 8, jedoch unter Verwendung eines handelsüblichen Harnstoff/Formaldenydharzes mit einem H:F-Verhältnis von 1:1,4 anstelle des Resols. Nach 5 Sekunden Bestrahlung erhält man einen klebfreien Ueberzug.

**BEISPIEL 10**

Ein Gemisch aus 96 Teilen eines handelsüblichen methylierten Melamin/Formaldehydharzes (im wesentlichen Hexamethoxymethylmelamin) und 4 Teilen Dimethyl-p-toluolsulfonylmethylsulfoxoniumhexafluorophosphat bringt man als 10 µm dicken Film auf Weissblech auf. Der Film wird unter den in Beispiel 5 beschriebenen Bedingungen 30 Sekunden lang bestrahlt und dann 15 Minuten auf 120°C erhitzt. Man erhält einen hochlösungsmittelfesten Ueberzug.

**BEISPIEL 11**

Eine aus 97 Teilen 2,2-Bis-(p-Glycidyloxyphenyl)-propan und 3 Teilen Dimethylsulfonylmethylsulfoxoniumhexafluorophosphat bestehende Zusammensetzung bringt man als 10 µm dicken Film auf Weissblech auf und bestrahlt wie in Beispiel 5 beschrieben. Nach 20 Sekunden erhält man einen klebfreien Ueberzug.

**BEISPIEL 12**

Man löst Tris-(dimethyl-p-toluolsulfonylmethylsulfoxonium)-orthophosphat (3 Teile) in 10 Teilen des in Beispiel 9 verwendeten Harnstoff/Formaldehydharzes und bestrahlt einen 10 µm dicken Ueberzug der Lösung auf Weissblech 10 Sekunden lang wie in Beispiel 5 beschrieben, wobei man einen klebfreien Ueberzug erhält.

**Patentansprüche:**

1. Aromatische Sulfonylsulfoxoniumsalze der Formel

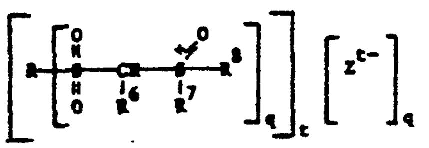

(VI),

worin q eine ganze Zahl von 1 bis 4 ist, R eine q-wertige aliphatische, cycloaliphatische oder aromatische Gruppe mit 1 bis 25 Kohlenstoffatomen bedeutet, die über eines ihrer Kohlenstoffatome direkt mit dem Schwefelatom der angegebenen benachbarten Sulfonylgruppe verknüpft ist, $R^6$ ein Wasserstoffatom, eine Alkyl- oder Aralkylgruppe mit 1 bis 25 Kohlenstoffatomen, eine Acylgruppe der Formel -$COR^9$ oder eine Gruppe der Formel

-CO-NH-$(CO)_r$-$R^{10}$ (VII)

oder

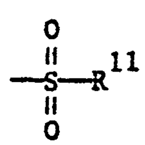

(VIII)

darstellt, wobei eines von R und $R^6$, aber nicht beide, für eine aromatische Gruppe mit 4 bis 25 Kohlenstoffatomen steht, $R^7$ eine Alkylgruppe mit 1 bis 12 Kohlenstoffatomen, eine Alkenylgruppe mit 2 bis 6 Kohlenstoffatomen, eine Cycloalkylgruppe mit 3 bis 8 Kohlenstoffatomen, eine Cycloalkylalkylgruppe mit 4 bis 10 Kohlenstoffatomen, eine Arylgruppe mit 4 bis 24 Kohlenstoffatomen oder eine Aralkylgruppe mit 5 bis 16 Kohlenstoffatomen bedeutet, $R^8$ dieselbe Bedeutung wie $R^7$ hat, aber auch für eine Dialkylaminogruppe mit 2 bis 6 Kohlenstoffatomen oder, falls $R^7$ Alkyl mit 1 bis 12 Kohlenstoffatomen darstellt, auch für eine Arylaminogruppe mit 4 bis 8 Kohlenstoffatomen stehen kann, $R^9$ einen Alkyl-, Cycloalkyl-, Aryloder Aralkylrest mit 1 bis 25 Kohlenstoffatomen bedeutet, der über eines seiner Kohlenstoffatome direkt an die angegebene -CO-Gruppe gebunden ist, r null oder 1 ist, $R^{10}$ für einen Alkyl-, Cycloalkyl-, Aryl- oder Aralkylrest mit 1 bis 25 Kohlenstoffatomen steht, der über eines seiner Kohlenstoffatome bei r= null an das angegebene Stickstoffatom oder bei r= 1 an das Kohlenstoffatom der angegebenen benachbarten Carbonylgruppe direkt gebunden ist, $R^{11}$ einen über eines seiner Kohlenstoffatome direkt an das angegebene Schwefelatom gebundenen Alkyl-, Cycloalkyl-, Aryl- oder Aralkylrest mit 1 bis 25 Kohlenstoffatomen darstellt, wobei $R^9$, $R^{10}$ und $R^{11}$ eine aromatische Gruppe bedeuten, wenn R eine aliphatische oder cycloaliphatische Gruppe ist, t für 1, 2 oder 3 steht und $Z^{t-}$ das t-wertige Anion einer Protonensäure bedeutet; wobei Dimethyl-p-toluolsulfonylmethylsulfoxoniumfluorid, Dimethyl-p-toluolsulfonylmethylsulfoxoniumchlorid, Dimethyl-p-methoxyphenylsulfonylmethylsulfoxoniumfluorid, Dimethyl-p-methoxyphenylsulfonylmethylsulfoxoniumchlorid, Dimethylbenzylsulfonylmethylsulfoxoniumfluorid und Dimethylbenzylsulfonylmethylsulfoxoniumchlorid ausgeschlossen sind.

2. Salze nach Anspruch 1, dadurch gekennzeichnet, dass R eine mono- oder dicyclische homocyclische Aryl- oder Aralkylgruppe mit 6 bis 16 Kohlenstoffatomen bedeutet.

3. Salze nach Anspruch 1, dadurch gekennzeichnet, dass R eine monocyclische oder dicyclische Arylen- oder Aralkylengruppe mit 6 bis 16 Kohlenstoffatomen bedeutet.

4. Salze nach einem der vorhergehenden Ansprüche, dadurch gekennzeichnet, dass $R^7$ und $R^8$ je für eine Alkylgruppe mit 1 bis 4 Kohlenstoffatomen oder eine gegebenenfalls in dem bzw. den aromatischen Ring(en) durch eine oder zwei Alkyl- oder Alkoxygruppen mit jeweils 1 bis 4 Kohlenstoffatomen oder ein oder zwei Fluor-, Chlor- oder Bromatome substituierte Phenyl- oder Naphthylgruppe stehen.

5. Salze nach einem der Ansprüche 1 bis 4, dadurch gekennzeichnet, dass R eine aromatische Gruppe und $R^9$ einen aliphatischen Rest mit 1 bis 8 Kohlenstoffatomen bedeuten.

6. Salze nach einem der Ansprüche 1 bis 5, dadurch gekennzeichnet, dass R eine aromatische Gruppe und $R^{10}$ einen aliphatischen Rest mit 1 bis 8 Kohlenstoffatomen bedeuten.

7. Salze nach einem der Ansprüche 1 bis 6, dadurch gekennzeichnet, dass R eine

aromatische Gruppe und $R^{11}$ einen aliphatischen Rest mit 1 bis 8 Kohlenstoffatomen bedeuten.

8. Salze nach einem der vorhergehenden Ansprüche, dadurch gekennzeichnet, dass $Z^{t-}$ das Anion einer anorganischen Säure bedeutet.

9. Salze nach einem der Ansprüche 1 bis 7, dadurch gekennzeichnet, dass $Z^{t-}$ $Cl^-$, $Br^-$, $NO_3^-$, $HSO_4^-$, $HSO_3^-$, $ClO_4^-$, $CF_3SO_3^-$, $CF_3COO^-$, $CH_3C_6H_4SO_3^-$, $H_2PO_4^-$, $SO_4^{--}$, $PO_4^{---}$, $SbF_5(OH)^-$ oder ein Anion der Formel

$MX_n^-$ (XI)

worin M für ein Antimon-, Wismut-, Bor-, Arsen- oder Phosphoratom und X für ein Fluor- oder Chloratom stehen sowie n 4, 5 oder 6 und um eins grösser als die Wertigkeit von M ist, bedeutet.

10. Salze nach Anspruch 1, dadurch gekennzeichnet, dass es sich um Dimethyl-p-toluolsulfonylmethylsulfoxonium-hexafluorphosphat, Dimethyl-p-toluolsulfonylmethylsulfoxonium-hexafluorantimonat, Tris-(dimethyl-p-toluolsulfonylmethylsulfoxonium)-orthophosphat und Dimethylsulfonylmethylsulfoxoniumhexafluorphosphat handelt.

11. Zusammensetzungen, dadurch gekennzeichnet, dass sie aus

(a) einer Verbindung bzw. einem Gemisch von Verbindungen, die unter dem Einfluss eines kationischen Katalysators in höhermolekulares Material überführbar sind, und

(b) einem aromatischen Sulfonylsulfoxoniumsalz der Formel VI bestehen

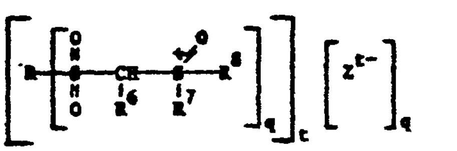

(VI),

worin q eine ganze Zahl von 1 bis 4 ist, R eine q-wertige aliphatische, cycloaliphatische oder aromatische Gruppe mit 1 bis 25 Kohlenstoffatomen bedeutet, die über eines ihrer Kohlenstoffatome direkt mit dem Schwefelatom der angegebenen benachbarten Sulfonylgruppe verknüpft ist, $R^6$ ein Wasserstoffatom, eine Alkyl- oder Aralkylgruppe mit 1 bis 25 Kohlenstoffatomen, eine Acylgruppe der Formel $-COR^9$ oder eine Gruppe der Formel

$-CO-NH-(CO)_r-R^{10}$ (VII)

oder

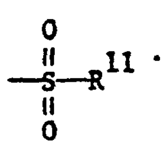

(VIII)

darstellt, wobei eines von R und $R^6$, aber nicht beide, für eine aromatische Gruppe mit 4 bis 25 Kohlenstoffatomen steht, $R^7$ eine Alkylgruppe mit 1 bis 12 Kohlenstoffatomen, eine Alkenylgruppe mit 2 bis 6 Kohlenstoffatomen, eine Cycloalkylgruppe mit 3 bis 8 Kohlenstoffatomen, eine Cycloalkylalkylgruppe mit 4 bis 10 Kohlenstoffatomen, eine Arylgruppe mit 4 bis 24 Kohlenstoffatomen oder eine Aralkylgruppe mit 5 bis 16 Kohlenstoffatomen bedeutet, $R^8$ dieselbe Bedeutung wie $R^7$ hat, aber auch für eine Dialkylaminogruppe mit 2 bis 6 Kohlenstoffatomen oder, falls $R^7$ Alkyl mit 1 bis 12 Kohlenstoffatomen darstellt, auch für eine Arylaminogruppe mit 4 bis 8 Kohlenstoffatomen stehen kann, $R^9$ einen Alkyl-, Cycloalkyl-, Aryloder Aralkylrest mit 1 bis 25 Kohlenstoffatomen bedeutet, der über eines seiner Kohlenstoffatome direkt an die angegebene -CO-Gruppe gebunden ist, r null oder 1 ist, $R^{10}$ für einen Alkyl-, Cycloalkyl-, Aryl- oder Aralkylrest mit 1 bis 25 Kohlenstoffatomen steht, der über eines seiner Kohlenstoffatome bei r= null an das angegebene Stickstoffatom oder bei r= 1 an das Kohlenstoffatom der angegebenen benschbarten Carbonylgruppe direkt gebunden ist, $R^{11}$ einen über eines seiner Kohlenstoffatome direkt an das sngegebene Schwefelatom gebundenen Alkyl-, Cycloalkyl-, Aryl- oder Aralkylrest mit 1 bis 25 Kohlenstoffatomen darstellt, wobei $R^9$, $R^{10}$ und $R^{11}$ eine aromatische Gruppe bedeuten, wenn R eine aliphatische oder cycloaliphatische Gruppe ist, t für 1, 2 oder 3 steht und $Z^t$ das t-wertige Anion einer Protonensäure bedeutet, wobei $Z^{t-}$ für $CF_3SO_3^-$ Gruppe $MX_n^-$ oder $SbF_5OH^-$ steht, falls (a) ein 1,2-Epoxid ist.

12. Zusammensetzungen nach Anspruch 11, dadurch gekennzeichnet, dass als (a) ein 1,2-Epoxid, ein Vinylmonomer oder -präpolymer, ein Aminoplast oder ein Phenoplast vorliegt.

13. Zusammensetzungen nach Anspruch 12, dadurch gekennzeichnet, dass als (a) entweder ein Epoxidharz oder ein aus einem Phenol und einem Aldehyd hergestelltes Resol vorliegt.

14. Zusammensetzungen nach einem der Ansprüche 11 bis 13, dadurch gekennzeichnet, dass sie 0,1 bis 7,5 Gewichtsteile (b) auf 100 Gewichtsteile (a) enthalten.

15. Zusammensetzungen nach Anspruch 13 oder 14, dadurch gekennzeichnet, dass sie ferner eine härtende Menge eines latenten Heisshärters für Epoxidharze oder Resolharze enthalten.

16. Verfahren zur Ueberführung einer Verbindung bzw. eines Gemischs von unter dem Einfluss eines kationischen Katalysators in höhermolekulares Material umwandelbaren Verbindungen in höhermolekulares Material, dadurch gekennzeichnet, dass man diese Verbindung bzw. dieses Gemisch in Gegenwart eines aromatischen Sulfonylsulfoxoniumsalzes nach Anspruch 11 aktinischer Strahlung und/oder Hitze unterwirft.

**Claims**

1. An aromatic sulfonylsulfoxonium salt of the formula

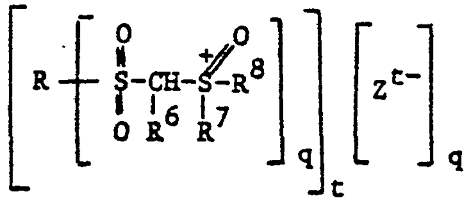

(VI)

wherein 9 is an integer from 1 to 4, R is a q-valent aliphatic, cycloaliphatic or aromatic group of 1 to 25 carbon atoms which is directly linked through a carbon atom thereof to the sulfur atom of the indicated adjacent sulfonyl group, $R^6$ is a hydrogen atom, an alkyl or aralkyl group of 1 to 25 carbon atoms, an acyl group of formula $-COR^9$ or a group of formula

$-CO-NH-(CO)_r-R^{10}$ (VII)

or

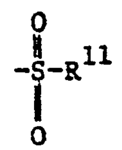

(VIII)

one, but not both, of R and $R^6$ being an aromatic group of 4 to 25 carbon atoms, $R^7$ is an alkyl group of 1 to 12 carbon atoms, an alkenyl group of 2 to 6 carbon atoms, a cycloalkyl group of 3 to 8 carbon atoms, a cycloalkylalkyl group of 4 to 10 carbon atoms, an aryl group of 4 to 24 carbon atoms, or an aralkyl group of 5 to 16 carbon atoms, $R^8$ has the same meaning as $R^7$ but may also be a dialkylamino group of 2 to 6 carbon atoms or, if $R^7$ is an alkyl group of 1 to 12 carbon atoms, $R_8$ may also be an arylamino group of 4 to 8 carbon atoms, $R^9$ is an alkyl, cycloalkyl, aryl or aralkyl radical of 1 to 25 carbon atoms which is directly linked through a carbon atom thereof to the indicated -CO- group, r is zero or 1, $R^{10}$ is an alkyl, cycloalkyl, aryl or aralkyl radical of 1 to 25 carbon atoms which is directly linked through a carbon atom thereof to, if r is zero, the indicated nitrogen atom,or to, if r is 1, the carbon atom of the indicated adjacent carbonyl group, $R^{11}$ is an alkyl, cycloalkyl, aryl or aralkyl radical of 1 to 25 carbon atoms which is directly linked through a carbon atom thereof to the indicated sulfur atom, each of $R^9$, $R^{10}$ and $R^{11}$ being an aromatic group if R is an aliphatic or cycloaliphatic group, t is 1, 2 or 3, and $Z^{t-}$ is the t-valent anion of a protonic acid; with the exception of

dimethyl-p-toluenesulfonylmethylsulfoxonium fluoride,

dimethyl-p-toluenesulfonylmethylsulfoxonium chloride,

dimethyl-p-methoxyphenylsulfonylmethylsulfoxonium fluoride,

dimethyl-p-methoxyphenylsulfonylmethylsulfoxonium chloride,

dimethylbenzylsulfonylmethylsulfoxonium fluoride and

dimethylbenzylsufonylmethylsulfoxonium chloride.

2. A salt according to claim 1, wherein R is a monocylic or dicyclic homocyclic aryl or aralkyl group of 6 to 16 carbon atoms.

3. A salt according to claim 1, wherein R is a monocyclic or dicyclic arylene or aralkylene group of 6 to 16 carbon atoms.

4. A salt according to any one of the preceding claims, wherein $R^7$ and $R^8$ are each an alkyl group of 1 to 4 carbon atoms, or a phenyl or a naphthyl group which may be substituted in the aromatic ring or rings by one or two alkyl groups, each of 1 to 4 carbon atoms, or by one or two alkoxy groups, each of 1 to 4 carbon atoms, or by one or two fluorine, chlorine, or bromine atoms.

5. A salt according to any one of claims 1 to 4, wherein R is an aromatic group and $R^9$ denotes an aliphatic radical of 1 to 8 carbon atoms.

6. A salt according to any one of claims 1 to 5, wherein R is an aromatic group and $R^{10}$ is an aliphatic radical of 1 to 8 carbon atoms.

7. A salt according to any one of claims 1 to 6, wherein R is an aromatic group and $R^{10}$ is an aliphatic radical of 1 to 8 carbon atoms.

8. A salt according to any one of the preceding claims, wherein $Z^t$ is an anion of an inorganic acid.

9. A salt according to any one of claims 1 to 7, wherein $Z^{t-}$ is $Cl^-$, $Br^-$, $NO_3^-$, $HSO_4^-$, $HSO_3^-$, $ClO_4^-$, $CF_3SO_3^-$, $CF_3COO^-$, $CH_3C_6H_4SO_3^-$, $H_2PO_4^-$, $SO_4^{--}$, $PO_4^{---}$, $SbF_5(OH)-$, or an anion of formula

$MX_n^-$ (XI)

wherein M is an antimony, bismuth, boron, arsenic or phosphorus atom, X is a fluorine or chlorine atom, and n is 4, 5, or 6 and is one more than the valency of M.

10. A salt according to claim 1, which is dimethyl-ptoluenesulfonylmethylsulfoxonium hexafluorophosphate, dimethyl-p-toluenesulfonylmethylsulfoxonium hexafluoroantimonate, tris(dimethyl-p-toluenesulfonylmethylsulfoxonium) orthophqsphate, or dimethylphenylsulfonylmethylsulfoxonium hexafluorophosphate.

11. A composition comprising

(a) a compound, or mixture of compounds, capable of being transformed into higher molecular weight-material under the influence of a cationic catalyst, and

(b) an aromatic sulfonylsulfoxonium salt of formula VI

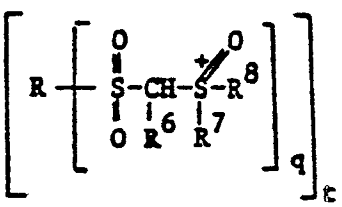

(VI)

wherein q is an integer from 1 to 4, R is a q-valent aliphatic, cycloaliphatic or aromatic group of 1 to 25 carbon atoms which is directly linked through a carbon atom thereof to the sulfur atom of the indicated adjacent sulfonyl group, $R^6$ is a hydrogen atom, an alkyl or aralkyl group of 1 to 25 carbon atoms, an acyl group of formula -COR or a group of formula

-CO-NH-(CO)$_r$-$R^{10}$ (VII)

or

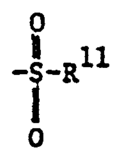

(VIII)

one, but not both, of R and $R^6$ being an aromatic group of 4 to 25 carbon atoms, $R^7$ is an alkyl group of 1 to 12 carbon atoms, an alkenyl group of 2 to 6 carbon atoms, a cycloalkyl group of 3 to 8 carbon atoms, a cycloalkylalkyl group of 4 to 10 carbon atoms, an aryl group of 4 to 24 carbon atoms, or an aralkyl group of 5 to 16 carbon atoms, $R^8$ has the same meaning as $R^7$ but may also be a dialkylamino group of 2 to 6 carbon atoms or, if $R^7$ is an alkyl group of 1 to 12 carbon atoms, $R_8$ may also be an arylamino group of 4 to 8 carbon atoms, $R^9$ is an alkyl,cycloalkyl, aryl or aralkyl radical of 1 to 25 carbon atoms which is directly linked through a carbon atom thereof to the indicated -CO- group, r is zero or 1, $R^{10}$ is an alkyl, cycloalkyl, aryl or aralkyl radical of 1 to 25 carbon atoms which is directly linked through a carbon atom thereof to, if r is zero, the indicated nitrogen atom,or to, if r is 1, the carbon atom of the indicated adjacent carbonyl group, $R^{11}$ is an alkyl, cycloalkyl, aryl or aralkyl radical of 1 to 25 carbon atoms which is directly linked through a carbon atom thereof to the indicated sulfur atom, each of $R^9$, $R^{10}$ and $R^{11}$ being an aromatic group if R is an aliphatic or cycloaliphatic group, t is 1, 2 or 3, and $Z^{t-}$ is the t-valent anion of a protonic acid; Z being $CF_3SO_3$ or an $MX_n^-$ or $SbF_5OH^-$ group if (a) is a 1,2-epoxide.

12. A composition according to claim 11, wherein (a) is a 1,2-epoxide, a vinyl monomer or prepolymer, an aminoplast, or a phenoplast.

13. A composition according to claim 12, wherein (a) is either an epoxide resin or a resol made from a phenol and an aldehyde.

14. A composition according to any one of claims 11 to 13, containing 0.1 to 7.5 parts by weight of (b) per 100 parts by weight of (a).

15. A composition according to either of claims 13 or 14, which also contains a curing amount of a latent heat-curing agent for epoxide resins or resol resins.

16. A process for the transformation into higher molecular weight-material of a compound, or mixture of compounds, capable of being converted into higher molecular weight-material under the influence of a cationic catalyst, comprising subjecting said compound or said mixture to actinic radiation and/or heat in the presence of an aromatic sulfonylsulfoxonium salt according to claim 11.

**Revendications**

1.- Sels de sulfonylsulfoxoniums aromatiques de formule

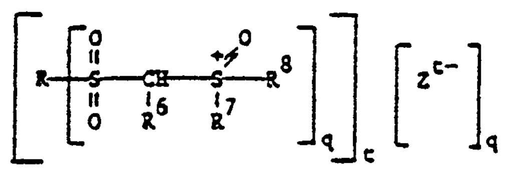

(VI)

dans laquelle q est un nombre entier de 1 à 4, R un groupe aliphatique, cycloaliphatique ou aromatique en $C_1$ à $C_{25}$, de valence q, directement lié par l'un de ses atomes de carbone à l'atome de soufre du groupe sulfonyle voisin, $R^6$ est un atome d'hydrogène, un alkyle ou un aralkyle en $C_1$ à $C_{25}$, un groupe acyle -$COR^9$ ou un groupe de formule

CO-NH-(CO)$_r$ -$R^{10}$ VII

ou

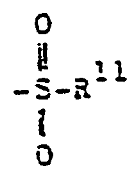

VIII

parmi R et $R^6$, l'un des deux mais pas les deux étant un groupe aromatique en $C_2$ à $C_{25}$, $R^7$ est un alkyle en $C_1$ à $C_{12}$, un alcényle en $C_2$ à $C_6$, un cycloalkyle en $C_3$ à $C_8$, un cycloalkylalkyle en $C_4$ à $C_{10}$, un aryle en en $C_4$ à $C_{24}$ ou un aralkyle en $C_5$ à $C_{16}$, $R^8$ a la même signification que $R^7$ mais peut être également un groupe dialkylamino en $C_2$ à $C_6$ ou bien, si $R^7$ est un alkyle en $C_1$ à $C_{12}$, peut être encore un groupe arylamino en $C_4$ à $C_8$, $R^9$ étant un alkyle, un cycloalkyle, un aryle ou un aralkyle en $C_1$ à $C_{25}$, directement lié au groupe -CO- par l'un de ses atomes de carbone, r est le nombre 0 ou 1, $R^{10}$ un alkyle, un cycloalkyle, un aryle ou un aralkyle en $C_1$ à $C_{25}$ directement lié à l'atome d'azote par l'un de ses atomes de carbone si r = 0, ou à l'atome de carbone du groupe carbonyle voisin si r = 1, $R^{11}$ étant un alkyle, un cycloalkyle,

un aryle ou un aralkyle en $C_1$ à $C_{25}$, directement lié à l'atome de soufre par 1 de ses atomes de carbone, $R^9$, $R^{10}$ et $R^{11}$ étant chacun un groupe aromatique si R est un groupe aliphatique ou cycloaliphatique, t est le nombre 1, 2 ou 3 et $Z^{t-}$ l'anion de valence t d'un acide protonique, à l'exclusion du fluorure et du chlorure de diméthyl-p-toluene-sulfonyl-méthylsulfoxonium, du fluorure et du chlorure de diméthyl-p-méthoxyphényl-sulfonylméthyl-sulfoxonium et du chlorure et du chlorure de diméthylbenzylsulfonyl-méthylsulfoxonium.

2.- Sels selon la revendication 1, caractérisés en ce que R est un groupe aryle ou aralkyle homocyclique monocyclique ou dicyclique, en $C_6$ à $C_{16}$.

3.- Sels selon la revendication 1, caractérisés en ce que R est un groupe arylène ou aralkylène monocyclique ou dicyclique en $C_2$ ü $C_{16}$.

4.- Sels selon l'une quelconque des revendications précédentes, caractérisés en ce que $R^7$ et $R^8$ sont chacun un alkyle en $C_1$ à $C_4$ ou bien un groupe méthyle ou naphtyle éventuellement substitué sur le ou les cycles aromatiques par un ou deux groupes alkyles ou alcoxy en $C_1$ à $C_4$ chacun ou par un ou deux atomes de fluor, de chlore ou de brome.

5.- Sels selon l'une quelconque des revendications 1 à 4, caractérisés en ce que R est un groupe aromatique et $R^9$ un groupe aliphatique en $C_1$ à $C_6$.

6.- Sels selon l'une quelconque des revendications 1 à 5, caractérisés en ce que R est un groupe aromatique et $R^{10}$ un groupe aliphatique en $C_1$ à $C_8$.

7. Sels selon l'une quelconque des revendications 1 à 6, caractérisés en ce que R est un groupe aromatique et $R^{11}$ un groupe aliphatique en $C_1$ à $C_8$.

8.- Sels selon l'une quelconque des revendications précédentes, caractérisés en ce que $Z^{t-}$ est l'anion d'un acide minéral.

9.- Sels selon l'une quelconque des revendications 1 à 7, caractérisés en ce que $Z^{t-}$ représente $Cl^-$, $Br^-$, $NO_3^-$ $HSO_4^-$, $HSO_3^-$, $C10_4^-$, $CF_3SO_3^-$, $CF_3COO^-$, $CH_3C_6H_4SO_3^-$, $H_2PO_4^-$, $SO_4^{--}$, $PO_4^{---}$, $SbF_5(OH)$ ou un anion de formule

$MX_n^-$ (XI)

dans laquelle M est un atome d'antimoine, de bismuth, de bore, d'arsenic ou de phosphore et X un atome de fluor ou de chlore, et n le nombre 4, 5 ou 6, supérieur d'une unité à la valence de M.

10.- Sels selon la revendication 1, comprenant l'hexafluoro-phosphate de diméthyl-p-toluene-sulfonylméthyl-sulfoxonium, l'hexafluoro-antimoniate de diméthylp-toluàne-sulfonylméthyl-sulfoxonium, l'orthophosphate de tris-(diméthyl-p-toluàne-sulfonylméthyl-sulfoxonium) et l'hexafluorophosphate de diméthylsulfonylméthyl-sulfoxonium.

11.- Compositions caractérisées en ce qu'elles comprennent:

(a) un composé ou un mélange de plusieurs composés transformables en matières à haute masse moléculaire sous l'effet d'un catalyseur cationique, avec

(b) un sel de sulfonyl-sulfoxonium aromatique de formule VI

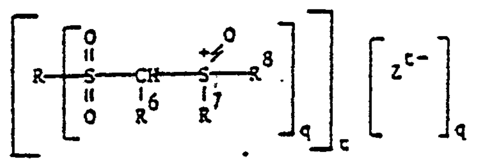
(VI)

dans laquelle q est un nombre entier de 1 à 4, R un groupe aliphatique, cycloaliphatique ou aromatique en $C_1$ à $C_{25}$, de valence q, directement lié par l'un de ses atomes de carbone à l'atome de soufre du groupe sulfonyle voisin, $R^6$ est un atome d'hydrogàne, un alkyle ou un aralkyle en $C_1$ à $C_{25}$, un groupe acyle $-COR^9$ ou un groupe de formule

$-CO-NH-(CO)_r-R^{10}$ (VII)

ou

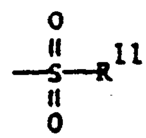
(VIII)

parmi R et $R^6$, l'un des deux mais pas les deux étant groupe aromatique en $C_2$ à $C_{25}$, $R^7$ est un alkyle en $C_1$ à $C_{12}$ un alcényle en $C_2$ à $C_6$, un cycloalkyle en $C_3$ à $C_8$, un cycloalkylalkyle en $C_4$ à $C_{10}$, un aryle en en $C_4$ à $C_{24}$ ou un aralkyle en $C_5$ à $C_{16}$, $R^8$ a la même signification que $R^7$ mais peut être également un groupe dialkylamino en $C_2$ ü $C_6$ ou bien, si $R^7$ est un alkyle en $C_1$ à $C_{12}$, peut être encore un groupe arylamino en $C_4$ à $C_8$, R étant un alkyle, un cycloalkyle, un aryle ou un aralkyle en $C_1$ à $C_{25}$, directement lié au groupe -CO- par l'un de ses atomes de carbone, r est le nombre 0 ou 1, $R^{10}$ un alkyle, un cycloalkyle, un aryle ou un aralkyle en $C_1$ à $C_{25}$, directement lié à l'atome d'azote par l'un de ses atomes de carbone si r = 0, ou à l'atome de carbone du groupe carbonyle voisin si r = 1, $R^{11}$ étant un alkyle, un cycloalkyle, un aryle ou un aralkyle en C à $C_{25}$ directement lié à l'atome de soufre par 'un de ses atomes de carbone $R^9$ $R^{10}$ et $R^{11}$ étant chacun un aliphatique, t est le nombre 1, 2 ou 3 et $Z^{t-}$ l'anion de valence t d'un acide protonique, $Z^{t-}$ étant $CF_3SO_3$ ou un groupe $MX_n$ ou $SbF_5$ $OH^-$ si la composante (a) est un 1,2-époxyde.

12.- Compositions selon la revendication 11, caractérisées en ce que la composante (a) est un 1,2-époxyde, un monomère ou un prépolymère vinylique, ou bien un aminoplaste ou un phénoplaste.

13.- Compositions selon la revendication 12, caractérisées en ce que la composante (a) est une résine époxyde ou un résol formé avec un phénol et un aldéhyde.

14.- Compositions selon l'une quelconque des

revendications 11 à 13, caractérisées en ce qu'ellescontiennent 0,1 à 7,5 parties en poids de la composante (b) pour 100 parties en poids de la composante (a).

15.- Compositions selon la revendication 13 ou 14, caractérisées en ce qu'elles contiennent une proportion durcissante d'un thermodurcisseur latent pour résines époxydes ou résols.

16.- Procédé pour transformer en matières à haute masse moléculaire un composé ou un mélange de composés transformables en matiàres à haute masse moléculaire sous l'effet d'un catalyseur cationique, procédé caractérisé en ce que l'on soumet à un rayonnement actinique et/ou à l'action de la chaleur ce composé ou le mélange de composés en présence d'un sel de-sulfonylsulfoxonium aromatique selon la revendication 11.